# EUROPÄISCHE PATENTANMELDUNG

(11) **EP 3 162 790 A1**
(43) Veröffentlichungstag der Anmeldung: **03.05.2017**
(21) Anmeldenummer: 15192306.7
(22) Anmeldetag: 30.10.2015
(51) Int. Cl.: C07C 213/00, C07C 215/48

(54) **HERSTELLUNGSVERFAHREN FÜR ISOPHORONAMINOALKOHOL (IPAA)**

(71) Anmelder: Evonik Degussa GmbH, 45128 Essen (DE)
(72) Erfinder: RÜFER, Dr., Alexander Martin, 45657 Recklinghausen (DE); RITTSTEIGER, Anne, 59399 Olfen (DE); SPYROU, Dr., Emmanouil, 46514 Schermbeck (DE); SCHNEIDER, Sven, 45711 Datteln (DE); SOWKA, Sabrina, 48249 Dülmen (DE); OTT, Denise, 45772 Marl (DE)

(57) **Zusammenfassung**

Vorliegende Erfindung betrifft ein verbessertes Verfahren zur Herstellung von 3-(Aminomethyl)-3,5,5-trimethylcyclohexanol, im folgenden Isophoronaminoalkohol (IPAA) genannt.

## Beschreibung

Vorliegende Erfindung betrifft ein verbessertes Verfahren zur Herstellung von 3-(Aminomethyl)-3,5,5-trimethylcyclohexanol aus Isophoronnitril (IPN), im folgenden Isophoronaminoalkohol (IPAA) genannt.

IPAA stellt ein wichtiges Zwischenprodukt in verschiedenen Anwendungsbereichen dar. So dient es beispielsweise als Vorstufe pharmakologischer Produkte, insbesondere im Bereich der Influenza-Prophylaxe (WO2011-095576). Weitere Anwendungen beinhalten u.a. den Einsatz in Polymeren, Korrosionschutzmittel und Stabilisatoren (DE1229078).

Die bisherigen Verfahren zeichnen sich dabei durch vergleichsweise komplizierte Reaktionsführung und niedrige Ausbeuten aus.

Pandey et al. (Indian Journal of Chemistry, Vol. 43B, 2004, 2705-2707) beschreibt eine Syntheseroute zu IPAA, ausgehend von Isophoronnnitril (IPN). Dabei wird über mehrere Stufen vorgegangen: Zunächst erfolgt die Bildung des korrespondierenden Amids über Hydrolyse unter basischen Bedingungen. Anschließend erfolgen die Reduktion der Ketofunktion mittels Cyanoborhydrids und die Reduktion des Amids mittels Lithiumaluminiumhydrid. Eine ähnliche Vorgehensweise ist in den Anmeldungen WO2011/094953 und WO2011/095576 beschrieben, hier erfolgt die Reduktion der Keto- und Nitrilgruppe von IPN jedoch ausschließlich mit Lithiumaluminiumhydrid in THF unter Rückfluss. Als nachteilig ist anzusehen, dass die o.g. Verfahren den stöchiometrischen Einsatz aufwendig herzustellender und damit teurer Reduktionsmittel vorsehen. Weiterhin erfolgt die Synthese über mehrere Stufen und ist damit aufwendig.

Die Anmeldung DE1229078 beschreibt die Herstellung von IPAA aus IPN unter Zuhilfenahme von Ammoniak und Wasserstoff an einem Fischer-Tropsch-Katalysator und sehr hohem Wasserstoffpartialdruck. Die hierbei erforderlichen, relativ hohen Reaktionstemperaturen begünstigen jedoch im Falle von gamma-Ketonitrilen (wie IPN) die Abspaltung von Blausäure (HCN). Dieser Umstand führt zur Ausbeuteminderung und ist daher nachteilig. Als weiterer Nachteil ist anzusehen, dass HCN stark toxisch auf Organismen wirkt (Gefahr bei Freisetzung) und üblicherweise zur Katalysatordesaktivierung beiträgt. Ferner erfolgt substanzielle Dimerenbildung aufgrund der Reaktion der entstehenden Amingruppe mit noch nicht vollständig reduzierter Ketogruppe. Dies stellt ebenso einen Ausbeuteverlust bezogen auf das Wunschprodukt IPAA dar.

Es ist auch bekannt aus EP 42 119, EP 659 734, EP 503 246, WO 2012076315, dass sich bei der konventionellen Hydrierung von IPN, das IPAA nur als Nebenprodukt in unerwünschter Weise bildet, was auch als Nachteil bezeichnet wird. Auch hier wird auf die Abspaltung von Blausäure (HCN) hingewiesen.

Daher sind im Stand der Technik keine geeigneten Katalysatoren zur gezielten einfachen Herstellung von IPAA aus IPN bekannt.

Aufgabe war es deshalb, ein neues verbessertes Verfahren zur gezielten Herstellung von 3-(Aminomethyl)-3,5,5-trimethylcyclohexanol, durch Hydrierung von Isophoronnnitril (IPN) mit Hilfe von Wasserstoff an einem Katalysator zu finden. Das Verfahren sollte sich durch eine einfache Durchführung und einer hohen Ausbeute auszeichnen. Außerdem muss die Abspaltung von Blausäure (HCN) vermieden werden.

Überraschenderweise wurde nun gefunden, dass eine einstufige Hydrierung von IPN mithilfe von Wasserstoff an einem heterogenen Katalysator mit einer einfachen einstufigen Fahrweise und einer sehr hohen Ausbeute möglich ist.

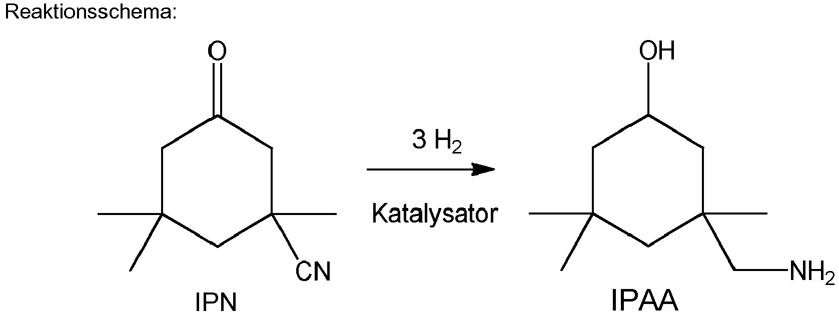

Gegenstand der Erfindung ist ein Verfahren zur einstufigen Herstellung von 3-(Aminomethyl)-3,5,5-trimethylcyclohexanol, durch Hydrierung von Isophoronnnitril (IPN) mit Wasserstoff an einem Katalysator, in An- oder Abwesenheit von Lösungsmittel,
wobei der Katalysator die folgenden Eigenschaften aufweist:
I.
Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |

und
II.
Der Katalysator liegt in Form von Hohlkugeln vor, mit Durchmessern von 1 bis 8 mm.

Das Katalysatormaterial besteht aus einer Metall-Legierung, wobei die Metall-Legierung durch Basen auf der Oberfläche aktiviert ist. Die Schichtdicke der aktivierten Schicht auf der Partikeloberfläche des Katalysators beträgt bevorzugt 50 bis 1500 Mikrometer (µm). Sie kann aber auch größer oder kleiner sein. Auf der Oberfläche befindet sich demnach die katalytisch aktive Zusammensetzung des Katalysators. Es ist aber auch im Rahmen der Erfindung möglich, das gesamte Katalysator-Partikel fast vollständig oder vollständig auszulaugen.

Der erfindungsgemäße Katalysator liegt nach der Aktivierung als Hohlkugeln vor.

Das erfindungsgemäße Katalysatormaterial weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
1. Variante

| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |

und/oder
2. Variante

| | |
|---|---|
| Cobalt: | 55 bis 90 Gew.-% |
| Aluminium: | 5 bis 44,5 Gew.-% |
| Chrom: | 0,5 bis 5 Gew.-% |

und/oder
3. Variante

| | |
|---|---|
| Cobalt: | 55 bis 88 Gew.-% |
| Aluminium: | 5 bis 44,5 Gew.-% |
| Nickel: | 0,5 bis 7 Gew.-% |

und/oder
4. Variante

| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |

und/oder
5. Variante

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

Gesamtheit bedeutet, dass bei der Zusammensetzung nicht zwischen dem Gehalt der Metalle auf der Oberfläche und in der aktivierten Schicht und im Kern der Katalysator-Partikel unterschieden wird, sondern Alles zusammen addiert und berechnet wird.

Der Katalysator liegt in Form von Hohlkugeln vor.

Zusätzlich weist der erfindungsgemäße Hohlkugel Katalysator nach der Aktivierung die folgenden Durchmesser auf:
Im Allgemeinen kann der Durchmesser des Katalysators, also die Hohlkugeln, Durchmesser von 1 bis 8 Millimeter (mm) aufweisen.

In einer ersten bevorzugten Variante der Erfindung variieren die Durchmesser des Katalysators, also die Hohlkugeln von 2,5 bis 5,5 Millimeter (mm).

In einer zweiten bevorzugten Variante der Erfindung variieren die Durchmesser des Katalysators, also die Hohlkugeln von 3 bis 8 Millimeter (mm).

In einer dritten bevorzugten Variante der Erfindung variieren die Durchmesser des Katalysators, also die Hohlkugeln von 1 bis 3 Millimeter (mm).

Das für die Herstellung des erfindungsgemäßen Katalysators verwendete Legierungspulver hat eine bevorzugte Korngröße von 5 bis 150 Mikrometer (µm). Die Korngröße kann aber auch kleiner oder größer gewählt werden.

Die Bestimmung der Partikelgrößen wird in der DIN ISO 9276-1 (September 2004) und 9276-2 (Februar 2006) und 9276-4 (Februar 2006) und 9276-6 (Januar 2012) beschrieben. Außerdem findet man genaue Angaben zur Definition von Partikelgrößen, der Verteilung von Partikelgrößen und der Messung von Partikelgrößen in HORIBA® Scientific, A GUIDEBOOK TO PARTICLE SIZE ANALYSIS, 2012, von HORIBA® Instruments, Inc, Irvine, USA.

Erfindungsgemäß kann die Verteilung der Partikelgrößen und die Messung der Partikelgrößen durch Laserverfahren (ISO 13320, 2012), Lichtverfahren oder Bildverfahren bestimmt werden.

Geeignete Methoden und Beschreibungen zur Siebanalyse sind beschrieben in:
DIN 66165-1:1987-04 Partikelgrößenanalyse; Siebanalyse; Grundlagen, und in DIN 66165-2:1987-04 Partikelgrößenanalyse; Siebanalyse; Durchführung.

Paul Schmidt, Rolf Körber, Matthias Coppers: Sieben und Siebmaschinen: Grundlagen und Anwendung. Wiley-VCH Verlag, 2003, ISBN 9783527302079, Kapitel 4.4: Analysesiebung. Jörg Hoffmann: Handbuch der Messtechnik. Hanser Verlag, 2007, ISBN 978-3-446-40750-3, Kapitel 3.12.16.2.1.

Der erfindungsgemäße Hohlkugel Katalysator weist besonders bevorzugt nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle (vierte bevorzugte Variante):

| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

und mit
Durchmesser des Hohlkugel Katalysators, also der Hohlkugeln mit einer statistischen Verteilung zwischen 2,5 bis 5,5 Millimeter (mm),
und/oder
Durchmesser des Hohlkugel Katalysators, also der Hohlkugeln mit einer statistischen Verteilung zwischen 3,5 bis 6,5 Millimeter (mm),
und/oder
Durchmesser des Hohlkugel Katalysators, also der Hohlkugeln mit einer statistischen Verteilung zwischen 1 bis 3 Millimeter (mm),
und/oder
Durchmesser des Hohlkugel Katalysators, also der Hohlkugeln mit einer statistischen Verteilung zwischen 3 bis 8 Millimeter (mm),
wobei bis zu 10 Prozent der Hohlkugeln auch außerhalb des genannten Bereichs der genannten Untergrenze oder Obergrenze, aber auch jeweils bis zu 10 Prozent außerhalb des genannten Bereichs der genannten Untergrenze und Obergrenze liegen können.

Der erfindungsgemäße Katalysator kann aus einer oder mehreren Schichten aufgebaut sein. Die Schichten können dabei sowohl aus dem gleichen, als auch aus unterschiedlichen Katalysatormaterialen, also Legierungspulvern, bestehen. Legierungspulver können sich dabei in der Zusammensetzung der Metalle und/oder der Korngröße unterscheiden. Durch die Verwendung von feinem Legierungspulver wird ein kompakter Schalenaufbau mit geringer Porosität und einer hohen mechanischen Stabilität erhalten. Grobes Legierungspulver dagegen ergibt einen porösen Aufbau der Hohlkugelschale. Diese führt zu einer Vergrößerung der aktiven Oberfläche der erfindungsgemäßen Hohlkugeln.

Über die Zusammensetzung des Legierungspulvers kann die Aktivität und/oder Selektivität des erfindungsgemäßen Katalysators beeinflusst werden.

Außerdem ist es möglich, dass der Hohlkugel förmige Katalysator eine bimodale oder multimodale Verteilung in Bezug auf die Durchmesser der Hohlkugeln aufweist. Bei einer bimodalen Verteilung werden demnach Hohlkugeln mit zwei verschiedenen Durchmessern eingesetzt, bei einer multimodalen Verteilung Hohlkugeln mit mindestens drei verschiedenen Durchmessern. Bevorzugt werden die Hohlkugeln gemäß der ersten bis dritten Variante und/oder Hohlkugeln gemäß der vierten Variante ausgewählt.

Außerdem ist es auch möglich, dass der Katalysator aus einer oder mehreren Schichten aufgebaut ist und/oder die Schichten dabei sowohl aus dem gleichen und/oder unterschiedlichen

Katalysatormaterialen, also Legierungspulvern, bestehen, und/oder, dass der Hohlkugelförmige Katalysator eine bimodale oder multimodale Verteilung in Bezug auf die Durchmesser der Hohlkugeln aufweist.

Der dieser Erfindung zugrunde liegende Vorteil wird durch die Verwendung von Katalysatoren in Form von Hohlkugeln erreicht. Die Herstellung der in dem erfindungsgemäßen Verfahren angewendeten Katalysatoren kann entsprechend der in DE 199 33 450.1 beschriebenen Methode durchgeführt werden. Nach dieser Methode wird eine Mischung eines Legierungspulvers aus katalytisch aktiven Metallen mit dem auslaugbaren Metall Aluminium, einem organischen Binder und gegebenenfalls einem anorganischen Binder, Wasser und Promotoren auf Kugeln, die aus einem thermisch zersetzbaren Material bestehen, aufgetragen. Bevorzugt können Polystyrolschaumkugeln verwendet werden. Das Auftragen der die Metall-Legierung enthaltenden Mischung auf die Polymerkugeln kann bevorzugt in einem Wirbelbett durchgeführt werden. Als organische Binder können bevorzugt 0 bis 10 Gew.-% Polyvinylalkohol und/oder 0 bis 3 Gew.-% Glycerin eingesetzt werden. Die beschichteten Polymerschaumkugeln werden anschließend oberhalb 300 °C, bevorzugt in einem Bereich zwischen 450 und 1300 °C calziniert, um den Polymerschaum thermisch zu entfernen und das Metall zu sintern. Dadurch erhalten die Hohlkugeln eine stabile Form. Nach der Calzinierung werden die Hohlkugelförmigen Katalysatoren durch Behandeln mit basischen Lösungen, bevorzugt Alkali- oder Erdalkalihydroxide in Wasser, noch bevorzugter wässrige Natronlauge aktiviert. Die so erhaltenen Katalysatoren besitzen Schüttdichten zwischen 0,3 und 1.3 kg/ I.

Erfindungsgemäß besitzen die in dem Verfahren angewendeten Katalysatoren die Form von Hohlkugeln. Hohlkugeln sind üblicherweise leicht herzustellen und besitzen eine hohe Bruchfestigkeit.

Die erfindungsgemäß angewendeten, Hohlkugel förmigen Katalysatoren können einen Binder enthalten. Der Binder ermöglicht eine größere Festigkeit der Katalysator Hohlkugel, die bedingt durch ihre hohle Form nötig ist. Bevorzugt werden Pulver der Metalle, die auch als katalytisch aktive Bestandteile in der Katalysatorlegierung enthalten sind, bei der Herstellung der Katalysator Hohlkugel als Binder zugesetzt. Es ist aber auch möglich, andere Binder, insbesondere andere Metalle als Binder zuzusetzen. Bevorzugt wird kein Binder eingesetzt. Hohlkugelförmige Kobaltkatalysatoren besitzen auch ohne zugesetzten Binder eine ausreichende Festigkeit. In dem erfindungsgemäßen Verfahren können mit anderen Metallen dotierte Hohlkugelförmige Raneykatalysatoren verwendet werden. Die Dotierungsmetalle werden oftmals auch als Promotoren bezeichnet. Das Dotieren von Raney- Katalysatoren wird beispielsweise in den Dokumenten US 4,153,578, DE 21 01 856, DE 21 00 373 oder DE 20 53 799 beschrieben. Bevorzugte Elemente zum Dotieren sind Elemente der Gruppen 1A, 2A, 3B bis 7B, 8, 1B, 2B und 3A des Periodensystems sowie Germanium, Zinn, Blei, Antimon und Wismut. Besonders bevorzugt sind Mangan, Eisen, Vanadium, Tantal, Titan, Wolfram, Molybdän, Rhenium und/oder Metalle der Platingruppe. Der Anteil an Promotoren im Katalysator kann bevorzugt 0 bis 5 Gew.-% betragen. Die Promotoren können bereits als Legierungsbestandteil enthalten sein, oder erst zu einem späteren Zeitpunkt, insbesondere nach der Aktivierung zugegeben werden.

Während der Herstellung des erfindungsgemäßen Katalysators ist eine oder mehrere Calzinierungen bei Temperaturen oberhalb von 300 °C, bevorzugt in einem Bereich zwischen 450 und 1300 °C erforderlich. Wird dieser Vorgang unter einer Sauerstoffhaltigen Atmosphäre durchgeführt, kann Sauerstoff in die Katalysatorschicht eingebunden werden. Der Anteil im Katalysator ist dabei von der Dauer der Calzinierung und/oder den Aktivierungsbedingungen abhängig und kann bevorzugt 0 bis 25 Gew.-% betragen.

Für den Fall, dass derartige Elemente enthalten sind, in einer Menge von maximal ca. 30 Gew.-%, reduziert sich der Anteil der oben genannten Metall Co und Al sowie gegebenenfalls Cr und Ni im Katalysator entsprechend, wobei die Anteile an Co und Al sowie gegebenenfalls Cr und Ni dann nach der Aktivierung sich zu mindestens 70 Gew.-% addieren, bezogen auf die enthaltenden Metalle.

In dem erfindungsgemäßen Verfahren werden Hohlkugelförmige Katalysatoren mit einem Durchmesser von 1 bis 8 mm und eine Schalendicke von 50 bis 1500 Mikrometer (µm) verwendet. Die Katalysatorschalen können undurchlässig sein, oder eine Porosität von 80% und höher aufweisen.

In dem erfindungsgemäßen Verfahren können Hohlkugelförmige Katalysatoren verwendet werden, die aus einer oder aus mehreren Schichten bestehen. Haben die Katalysatorkörper mehrere Schichten, werden die Katalysatorkörper bei der Herstellung zwischen den einzelnen Beschichtungsschritten getrocknet. Dieses wird bevorzugt im Wirbelbett bei Temperaturen von 60 bis 150 °C durchgeführt. Es ist auch möglich einen Hohlkugelförmigen Katalysator mit mehreren Schichten herzustellen, wobei nicht zwischen den einzelnen Beschichtungsschritten getrocknet wird.

Während des erfindungsgemäßen Verfahrens werden die Hohlkugelförmigen Katalysatoren in der aktivierten Form eingesetzt. Das in den nicht aktivierten Katalysatorkörpern vorhanden auslaugbare Metall kann im aktivierten Zustand ganz oder nur teilweise mit Alkalien herausgelaugt worden sein.

Allgemeine Methode zur Herstellung des Katalysators:

### a) Herstellung der Legierung

Die Herstellung der Legierung erfolgt thermisch, zum Beispiel in einem Induktionsofen. Es werden dabei die Metalle geschmolzen und eine Legierung erhalten. Die fertige Schmelze wird für die Weiterverarbeitung zum Beispiel zu Barren gegossen.

### b) Herstellung der Pulver

Die Legierung wird in geeigneten Geräten zu Pulver verarbeitet, zum Beispiel über einen Backenbrecher vorzerkleinert und über eine Kugel- oder Stabmühle weiter gemahlen. Durch einen optionalen Siebschritt kann die gewünschte Größenverteilung der Partikel durch die Wahl der entsprechenden Siebe erhalten werden.

### c) Herstellung der Hohlkugeln

Für die Herstellung der Hohlkugeln wird eine Mischung aus Legierungspulver, einem organischen und gegebenenfalls anorganischen Binder, Wasser und Promotoren auf Kugeln, die aus einem thermisch zersetzbaren Material bestehen, aufgetragen. Bevorzugt können Polystyrolschaumkugeln verwendet werden. Das Auftragen der die Metall-Legierung enthaltenden Mischung auf die Polymerkugeln kann bevorzugt in einem Wirbelbett durchgeführt werden. Als organische Binder können bevorzugt 0 bis 10 Gew.-% Polyvinylalkohol und/oder 0 bis 3 Gew.-% Glycerin eingesetzt werden. Die beschichteten Polymerschaumkugeln werden anschließend oberhalb von 300 °C, bevorzugt in einem Bereich zwischen 450 und 1300 °C calziniert, um den Polymerschaum thermisch zu entfernen und das Metall zu sintern. Dadurch erhalten die Hohlkugeln eine stabile Form.

### d) Aktivierung des Katalysators

Die Aktivierung des Katalysators erfolgt in geeigneten Apparaturen. Es können dabei organische oder anorganische Basen eingesetzt werden. Bevorzugt wird eine Lauge (z. B. Natronlauge), verwendet, wobei durch einen exothermen Vorgang ein Teil des Aluminiums unter Bildung von Wasserstoff und Aluminatlauge aus der Legierung herausgelöst wird. Die Konzentration der Lauge kann zwischen 5 und 30 Gew.-% liegen und die Reaktionstemperatur zwischen 50 und 110 °C. Der Grad der Aktivierung wird über die Temperatur und die Reaktionszeit bestimmt. Dabei ist die Reaktionszeit variabel und ist abhängig von den Reaktionsbedingungen und dem gewünschten Grad der Aktivierung. Nach der Aktivierung wird der Katalysator zur Entfernung des Aluminats mit kalter Lauge, danach mit Wasser gewaschen und anschließend unter Wasser gelagert. Andere Zusammensetzungen können im Herstellungsschritt a) durch die entsprechende Wahl der Metallmengen analog produziert werden.

Bevorzugt wird der Katalysator in der beschriebenen Reihenfolge hergestellt. Die Aktivierung des Katalysators kann aber auch vor der Herstellung der Hohlkugeln erfolgen.

### Durchführung der Hydrierung allgemein

Die Hydrierung erfolgt einstufig in Batch-Autoklaven oder Festbettreaktoren. Geeignete Reaktortypen sind z. B. Schachtöfen, Hordenreaktoren oder Rohrbündelreaktoren.

Der Prozess kann in diskontinuierlicher oder kontinuierlicher Fahrweise durchgeführt werden.

Die Hydrierung erfolgt üblicherweise bei Temperaturen zwischen 20 bis 200°C, bevorzugt 20 bis 150 °C, besonders bevorzugt 40 bis 130 °C, und Drücken von 0,3 bis 50 MPa, bevorzugt 0,3 bis 30 MPa, besonders bevorzugt 5 bis 15 MPa. Die Bereiche für Druck und Temperatur sind beliebig kombinierbar. Die Hydrierung kann in An- oder Abwesenheit von Lösungsmittel durchgeführt werden. Als Lösungsmittel eignen sich u.a. Ether, cyclische und lineare Kohlenwasserstoffe und Alkohole, bevorzugt werden Methanol, Ethanol oder THF verwendet.

Der für die Hydrierung erforderliche Wasserstoff kann dem Reaktor entweder im Überschuss, beispielsweise mit bis zu 10000 Moläquivalenten, zugeführt werden, oder nur in einer solchen Menge, dass der durch Reaktion verbrauchte Wasserstoff sowie der Teil des Wasserstoffs, der eingelöst im Produktstrom den Reaktor verlässt, nachgeführt wird. Bei kontinuierlicher Fahrweise kann der Wasserstoff im Gleich- oder Gegenstrom zugeführt werden.

Das erforderliche Volumen der einzusetzenden Hydrierkatalysatoren richtet sich nach dem vom Betriebsdruck, der Temperatur, der Konzentration und der Katalysatoraktivität abhängigen LHSV-Wert, (liquid hourly space velocity) der eingehalten werden muss, um eine möglichst vollständige Hydrierung des eingesetzten IPN zu gewährleisten. Üblicherweise liegt der LHSV-Wert bei der Verwendung des bevorzugt einzusetzenden Gemisches aus IPN und Wasserstoff zwischen 0,1 und 8 pro Liter Katalysator und Stunde, bevorzugt zwischen 1 und 4 I_{Lsg} I_{Kat}⁻¹ h⁻¹.

Bevorzugt wird die Hydrierung kontinuierlich in Festbettreaktoren durchgeführt, besonders bevorzugt kontinuierlich in Festbettreaktoren, die in Rieselbettfahrweise oder Sumpffahrweise betrieben werden.

Das die Hydrierung verlassende Reaktionsgemisch wird mit den üblichen Methoden weiter aufgereinigt, um ein IPAA mit der gewünschten Qualität zu erhalten. Dabei können alle gängigen Trennmethoden wie z. B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich, absatzweise, ein- oder mehrstufig, im Vakuum oder unter Druck durchgeführt werden.

Bevorzugt wird die Aufreinigung durch Destillation unter Druck und/oder im Vakuum in mehreren Schritten erreicht. Hierfür lassen sich beliebige Destillationskolonnen mit und ohne Einbauten wie z. B. Dephlegmatoren, Trennwänden, ungeordnete Einbauten oder Schüttungen, geordnete Einbauten oder Packungen, Böden mit oder ohne Zwangsführung verwenden.

### Beispiele

### Katalysatorherstellung

### Herstellung der Legierung

Die Herstellung der Legierung erfolgte in einem Induktionsofen. Es wurden dabei die Metalle in den entsprechenden Mengen bei 1500°C geschmolzen. Die fertige Schmelze wurde für die Weiterverarbeitung zu Barren gegossen.

### Herstellung der Pulver

Die Legierungsbarren wurden über einen Backenbrecher vorzerkleinert und über eine Kugelmühle weiter gemahlen. Durch einen Siebschritt wurde die gewünschte Größenverteilung des Pulvers durch die Wahl der entsprechenden Siebe erhalten.

### Beispiel 1: Herstellung der Hohlkugeln

Durch Suspendieren von 3179 g CoAlCrNi Legierung (Korngrößenverteilung < 200 µm) in 2861 g einer wässrigen Lösung mit einem Gehalt an ca. 2 Gew.-% Polyvinylalkolhol wurde eine Beschichtungslösung hergestellt. Diese Suspension wurde anschließend auf 1500 ml Polystyrolkugeln mit einem Durchmesser um ca. 1,8 mm aufgesprüht, während diese in einem nach oben gerichteten Luftstrom suspendiert waren.

1,5 Liter dieser Kugeln wurden mit einer Legierungslösung bestehend aus 3169 g CoAlCrNi Legierung suspendiert in 2852 g einer wässrigen Lösung mit einem Gehalt an ca. 2 Gew.-% Polyvinylalkolhol weiter beschichtet.

Nach dem Beschichten der Polystyrolkugeln mit den zuvor erwähnten Lösungen wurden die Kugeln bis 500 °C erwärmt, um das Polystyrol herauszubrennen. Die CoAlCrNi Hohlkugeln wurden anschließend bis 900°C erwärmt.

Die Hohlkugeln wurden nach Abkühlen in einer 20 Gew.-%igen Natronlauge 70 Minuten bei 90°C aktiviert. Die erhaltenen aktivierten Hohlkugeln hatten einen Durchmesser von 2,5 bis 5,5 mm und eine Manteldicke von 600 bis 1000 µm.

Der eingesetzte Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:

| | |
|---|---|
| Cobalt: | 55,6 Gew.-% |
| Aluminium: | 20,9 Gew.-% |
| Chrom: | 1,1 Gew.-% |
| Nickel: | 1,7 Gew.-% |
| Sauerstoff | 20,8 Gew.-% |

### Beispiel 2:

Der erfindungsgemäße Katalysator wurde bei der Herstellung von 3-Aminomethyl-3,5,5-Trimethylcyclohexanol (Isophoronaminoalkohol, IPAA) aus 3-Cyano-3,5,5-Trimethylcyclohexanon (Isophoronnitril, IPN) in einem diskontinuierlichen Verfahren in einem Laborautoklaven auf seine katalytische Wirksamkeit getestet.

Für die Durchführung der einzelnen Experimente wurde die Reaktionslösung (5 Gew.-% IPN in 95 Gew.-% THF) im Reaktor vorgelegt. 150ml des Katalysators befanden sich dabei in einem speziellen Katalysatorkorb. Nach dem Aufheizen auf die gewünschte Reaktionstemperatur wurde der gewünschte Reaktionsdruck durch Zugabe von Wasserstoff eingestellt. Die Untersuchung der Hydrierung von IPN mit den erfindungsgemäßen Katalysatoren erfolgte bei einer Temperatur von 60 und 80 °C bei einem Druck von 50 und 100 bar.

Die Reaktionsmischung wurde auf IPN, IPAA und entsprechende Nebenkomponenten gaschromatographisch untersucht. Die Ergebnisse sind in Tabelle 1 aufgeführt.

**Tabelle 1**

| Temperatur | Druck | Reaktionszeit | Ausbeute IPAA / GC-% | Umsatz / % |
|---|---|---|---|---|
| 60°C | 50 bar | 6h | 94,3 | 100 |
| 60°C | 100 bar | 5h | 96,2 | 100 |
| 80°C | 50 bar | 5h | 95,3 | 100 |

### Beispiel A (Vergleichsbeispiel):

Die Vergleichsbeispiele mit einem kommerziellen Referenzkatalysator wurden analog zur Beschreibung in Beispiel 2 durchgeführt. Als Referenzkatalysator wurden 150ml eines kommerziellen, geträgerten Cobalt-Fischer-Tropsch-Katalysatorformkörpers (Pellets) eingesetzt. Die Reaktionsmischung wurde auf IPN, IPAA und entsprechende Nebenkomponenten gaschromatographisch untersucht. Die Ergebnisse sind in Tabelle 2 aufgeführt.

**Tabelle 2**

| Temperatur | Druck | Reaktionszeit | Ausbeute IPAA / GC-% | Umsatz / % |
|---|---|---|---|---|
| 60°C | 50 bar | 5h | 40,4 | 85,1 |
| 60°C | 100 bar | 5h | 43,1 | 95,4 |
| 80°C | 50 bar | 5h | 44,2 | 98,2 |

Wie der Fachmann anhand der Tabelle 2 erkennen kann, ist die Ausbeute im Falle des kommerziellen Referenzkatalysators bei ähnlicher Reaktionszeit deutlich geringer, ebenso wie die erzielten Umsätze.

## Patentansprüche

1. Verfahren zur einstufigen Herstellung von 3-(Aminomethyl)-3,5,5-trimethylcyclohexanol, durch Hydrierung von IPN mit Wasserstoff an einem Katalysator, in An- oder Abwesenheit von Lösungsmittel,
wobei der Katalysator die folgenden Eigenschaften aufweist:
I.
Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |
und
II.
Der Katalysator liegt in Form von Hohlkugeln vor mit Durchmessern von 1 bis 8 mm.

2. Verfahren zur Herstellung von 3-(Aminomethyl)-3,5,5-trimethylcyclohexanol nach Anspruch 1, **dadurch gekennzeichnet, dass**
I.
Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 55 bis 90 Gew.-% |
| Aluminium: | 5 bis 44,5 Gew.-% |
| Chrom: | 0,5 bis 5 Gew.-% |

3. Verfahren zur Herstellung von 3-(Aminomethyl)-3,5,5-trimethylcyclohexanol nach Anspruch 1, **dadurch gekennzeichnet, dass**
I.
Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 55 bis 88 Gew.-% |
| Aluminium: | 5 bis 44,5 Gew.-% |
| Nickel: | 0,5 bis 7 Gew.-% |

4. Verfahren zur Herstellung von 3-(Aminomethyl)-3,5,5-trimethylcyclohexanol nach Anspruch 1, **dadurch gekennzeichnet, dass**
I.
Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 55 bis 85 Gew.-% |
| Aluminium: | 5 bis 43,5 Gew.-% |
| Chrom: | 0,5 bis 3 Gew.-% |
| Nickel: | 1 bis 7 Gew.-% |

5. Verfahren zur Herstellung von 3-(Aminomethyl)-3,5,5-trimethylcyclohexanol nach Anspruch 1, **dadurch gekennzeichnet, dass**
I.
Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |

6. Verfahren zur Herstellung von 3-(Aminomethyl)-3,5,5-trimethylcyclohexanol nach mindestens einem der vorherigen Ansprüche 1-5, **dadurch gekennzeichnet, dass**
der Durchmesser des Katalysators, also der Hohlkugeln, von 2,5 bis 5,5 Millimeter (mm) variiert,
und/oder
der Durchmesser des Katalysators, also der Hohlkugeln, von 3 bis 8 Millimeter (mm) variiert,
und/oder
die Durchmesser des Katalysators, also der Hohlkugeln, von 1 bis 3 Millimeter (mm) variiert.

7. Verfahren zur Herstellung von 3-(Aminomethyl)-3,5,5-trimethylcyclohexanol nach Anspruch 1, **dadurch gekennzeichnet, dass** der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
| | |
|---|---|
| Cobalt: | 57 bis 84 Gew.-% |
| Aluminium: | 10 bis 40 Gew.-% |
| Chrom: | 1 bis 2 Gew.-% |
| Nickel: | 2 bis 4 Gew.-% |
und mit
Durchmessern des Hohlkugel Katalysators, also der Hohlkugeln mit einer statistischen Verteilung zwischen 2,5 bis 5,5 Millimeter (mm),
und/oder
Durchmessern des Hohlkugel Katalysators, also der Hohlkugeln mit einer statistischen Verteilung zwischen 3,5 bis 6,5 Millimeter (mm),
und/oder
Durchmessern des Hohlkugel Katalysators, also der Hohlkugeln mit einer statistischen Verteilung zwischen 1 bis 3 Millimeter (mm),
und/oder
Durchmessern des Hohlkugel Katalysators, also der Hohlkugeln mit einer statistischen Verteilung zwischen 3 bis 8 Millimeter (mm),
wobei bis zu 10 Prozent der Hohlkugelnauch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

8. Verfahren zur Herstellung von 3-(Aminomethyl)-3,5,5-trimethylcyclohexanol nach mindestens einem der vorherigen Ansprüche, **dadurch gekennzeichnet, dass** die Katalysatoren zusätzlich Dotiermetalle enthalten, bevorzugt ausgewählt aus Mangan, Eisen, Vanadium, Tantal, Titan, Wolfram, Molybdän, Rhenium und/oder Metalle der Platingruppe.

9. Verfahren zur Herstellung von 3-(Aminomethyl)-3,5,5-trimethylcyclohexanol nach mindestens einem der vorherigen Ansprüche, **dadurch gekennzeichnet, dass** der Anteil an Dotiermetallen im Katalysator 0 bis 5 Gew.-% beträgt.

10. Verfahren zur Herstellung von 3-(Aminomethyl)-3,5,5-trimethylcyclohexanolnach mindestens einem der vorherigen Ansprüche, **dadurch gekennzeichnet, dass** der Anteil an Sauerstoff im Katalysator 0 bis 25 Gew.-% beträgt.

11. Verfahren zur Herstellung von 3-(Aminomethyl)-3,5,5-trimethylcyclohexanolnach mindestens einem der vorherigen Ansprüche, **dadurch gekennzeichnet, dass** der Katalysator aus einer oder mehreren Schichten aufgebaut ist und/oder die Schichten dabei sowohl aus dem gleichen und/oder unterschiedlichen Katalysatormaterialen, also Legierungspulvern, bestehen, und/oder, dass der Hohlkugelförmige Katalysator eine bimodale oder multimodale Verteilung in Bezug auf die Durchmesser der Hohlkugeln aufweist.

12. Verfahren zur Herstellung von 3-(Aminomethyl)-3,5,5-trimethylcyclohexanol nach mindestens einem der vorherigen Ansprüche, **dadurch gekennzeichnet, dass** die Hydrierung bei einer Temperatur von 20 bis 200°C und bei einem Druck von 0,3 bis 50 MPa durchgeführt wird.

13. Verfahren zur Herstellung von 3-(Aminomethyl)-3,5,5-trimethylcyclohexanol nach mindestens einem der vorherigen Ansprüche, **dadurch gekennzeichnet, dass** die Hydrierung bei einer Temperatur von 40 bis 130 °C erfolgt.

14. Verfahren zur Herstellung von 3-(Aminomethyl)-3,5,5-trimethylcyclohexanol nach mindestens einem der vorherigen Ansprüche, **dadurch gekennzeichnet, dass** die Hydrierung bei Drücken von 5 bis 30 MPa erfolgt.

15. Verfahren zur Herstellung von 3-(Aminomethyl)-3,5,5-trimethylcyclohexanol nach mindestens einem der vorherigen Ansprüche, **dadurch gekennzeichnet, dass** die Hydrierung bei Drücken von 5 bis 15 MPa erfolgt.

16. Verfahren zur Herstellung von 3-(Aminomethyl)-3,5,5-trimethylcyclohexanol nach mindestens einem der vorherigen Ansprüche, **dadurch gekennzeichnet, dass** die Hydrierung einstufig in Batch-Autoklaven oder Festbettreaktoren erfolgt.

17. Verfahren zur Herstellung von 3-(Aminomethyl)-3,5,5-trimethylcyclohexanol nach mindestens einem der vorherigen Ansprüche, **dadurch gekennzeichnet, dass** die Hydrierung einstufig in Schachtöfen, Hordenreaktoren oder Rohrbündelreaktoren durchgeführt wird.

18. Verfahren zur Herstellung von 3-(Aminomethyl)-3,5,5-trimethylcyclohexanol nach mindestens einem der vorherigen Ansprüche, **dadurch gekennzeichnet, dass** der für die Hydrierung erforderliche Wasserstoff dem Reaktor entweder im Überschuss, bevorzugt mit bis zu 10000 Moläquivalenten, zugeführt wird, oder in einer solchen Menge, dass der durch Reaktion verbrauchte Wasserstoff sowie der Teil des Wasserstoffs, der eingelöst im Produktstrom den Reaktor verlässt, nachgeführt wird.

19. Verfahren zur Herstellung von 3-(Aminomethyl)-3,5,5-trimethylcyclohexanol nach mindestens einem der vorherigen Ansprüche, **dadurch gekennzeichnet, dass** Hydrierung kontinuierlich in Festbettreaktoren erfolgt.

20. Verfahren zur Herstellung von 3-(Aminomethyl)-3,5,5-trimethylcyclohexanol nach mindestens einem der vorherigen Ansprüche, **dadurch gekennzeichnet, dass** die Hydrierung kontinuierlich in Festbettreaktoren, die in Rieselbettfahrweise oder Sumpffahrweise betrieben werden, durchgeführt wird.

21. Verfahren zur Herstellung von 3-(Aminomethyl)-3,5,5-trimethylcyclohexanol nach mindestens einem der vorherigen Ansprüche, **dadurch gekennzeichnet, dass** das die Hydrierung verlassende Reaktionsgemisch einstufig oder mehrstufig aufgereinigt wird, und das 3-(Aminomethyl)-3,5,5-trimethylcyclohexanolerhalten wird.

22. Verwendung eines Katalysators zur Herstellung von 3-(Aminomethyl)-3,5,5-trimethylcyclohexanol,
wobei der Katalysator die folgenden Eigenschaften aufweist:
I.
Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
| | |
|---|---|
| Cobalt: | 55 bis 95 Gew.-% |
| Aluminium: | 5 bis 45 Gew.-% |
| Chrom: | 0 bis 3 Gew.-% |
| Nickel: | 0 bis 7 Gew.-% |
und
II.
Der Katalysator liegt in Form von Hohlkugeln vor mit Durchmessern von 1 bis 8 mm.
